Europäisches Patentamt

European Patent Office

Office européen des brevets

(11) Publication number: **0 372 993**
**A1**

## (12) EUROPEAN PATENT APPLICATION

(21) Application number: 89312832.2

(22) Date of filing: 08.12.89

(51) Int. Cl.5: **C07C 53/08, C07C 51/487**

(30) Priority: 09.12.88 US 282180

(43) Date of publication of application:
**13.06.90 Bulletin 90/24**

(84) Designated Contracting States:
**AT BE CH DE ES FR GB GR IT LI LU NL SE**

(71) Applicant: HOECHST CELANESE
CORPORATION
Route 202-206 North
Somerville, New Jersey(US)

(72) Inventor: Torrence, G Paul
301 South Morningside
Corpus Christi Texas(US)
Inventor: Colling, Philip M.
609 Barracuda
Corpus Christi Texas(US)
Inventor: Scates, Mark O.
1801 Crooked Creek
Pearland , Texas(US)
Inventor: Picard, wayne D.
16018 Havenhurst
Houston Texas(US)

(74) Representative: De Minvielle-Devaux, Ian
Benedict Peter et al
CARPMAELS & RANSFORD 43, Bloomsbury
Square
London WC1A 2RA(GB)

(54) Treatment of acetic acid with hydrogen in the presence of a hydrogenation catalyst.

(57) Acetic acid, containing iodide, unsaturate or carbonyl impurities or mixtures thereof, is subject to hydrogenation with hydrogen in the presence of a hydrogenation catalyst to obtain an acetic acid product having an improved quality as determined by the permanganate time.

EP 0 372 993 A1

# TREATMENT OF ACETIC ACID WITH HYDROGEN IN THE PRESENCE OF A HYDROGENATION CATALYST

## CROSS REFERENCE TO RELATED APPLICATIONS

Pending application Ser. No. 936,188 filed December 1, 1986, discloses purification of acetic acid by treatment with a compound such as hydrazine or derivatives thereof. Pending application Ser. No. 137,844, filed December 23, 1987, discloses a method of treating acetic acid to improve permanganate time by treatment with ozone.

## BACKGROUND OF THE INVENTION

### Field of the Invention

This invention relates to the treatment of acetic acid and relates more particularly to the treatment of acetic acid resulting from the catalytic carbonylation of methanol.

## DESCRIPTION OF THE PRIOR ART

Various methods have been employed for producing acetic acid including, for example, the oxidation of acetaldehyde, the oxidation of petroleum naphtha, butane or the like, or the direct synthesis from methanol and carbon monoxide. One of the more useful commercial methods for the production of acetic acid is the carbonylation of methanol as disclosed in U.S. 3,769,329. The carbonylation catalyst comprises rhodium, either dissolved or otherwise dispersed in a liquid reaction medium or else supported on an inert solid, along with a halogen-containing catalyst promoter as exemplified by methyl iodide. The rhodium can be introduced into the reaction system in any of many forms, and it is not relevant, if indeed it is possible, to identify the exact nature of the rhodium moiety within the active catalyst complex. Likewise, the nature of the halide promoter is not critical. A large number of suitable promoters are disclosed, most of which are organic iodides. Typically, the reaction is conducted with the catalyst being dissolved in a liquid reaction medium through which carbon monoxide gas is continuously bubbled.

An improvement in the prior-art process for the carbonylation of an alcohol to produce the carboxylic acid having one carbon atom more than the alcohol in the presence of a rhodium catalyst is disclosed in copending, commonly assigned application U.S. Serial No. 699,525, filed February 8, 1985 and European patent application 161,874; published November 21, 1985. As disclosed therein, acetic acid (HAc) is produced from methanol (MeOH) in a reaction medium comprising methyl acetate (MeOAc), methyl halide, especially methyl iodide, (MeI), and rhodium present in a catalytically-effective concentration. The invention therein resides primarily in the discovery that catalyst stability and the productivity of the carbonylation reactor can be maintained at surprisingly high levels, even at very low water concentrations, i.e. 4 wt.% or less, in the reaction medium (despite the general industrial practice of maintaining approximately 14 wt.% or 15 wt.% water) by maintaining in the reaction medium, along with a catalytically-effective amount of rhodium, at least a finite concentration of water, methyl acetate and methyl iodide, a specified concentration of iodide ions over and above the iodide content which is present as methyl iodide or other organic iodide. The iodide ion is present as a simple salt, with lithium iodide being preferred. The applications teach that the concentration of methyl acetate and iodide salts are significant parameters in affecting the rate of carbonylation of methanol to produce acetic acid especially at low reactor water concentrations. By using relatively high concentrations of the methyl acetate and iodide salt, one obtains a surprising degree of catalyst stability and reactor productivity even when the liquid reaction medium contains water in concentrations as low as about 0.1 wt.%, so low that it can broadly be defined simply as "a finite concentration" of water. Furthermore, the reaction medium employed improves the stability of the rhodium catalyst, i.e. resistance to catalyst precipitation, especially during the product-recovery steps of the process wherein distillation for the purpose of recovering the acetic acid product tends to remove from the catalyst the carbon monoxide which in the environment maintained in the reaction vessel, is a ligand with stabilizing effect on the rhodium. U.S. Serial No. 699,525 is herein incorporated by reference.

The acetic acid which is formed by the carbonylation of methanol is converted to a high purity product by conventional means such as by a series of distillations. While it is possible in this manner to obtain acetic acid of relatively high purity, the acetic acid product contains some by-product impurities, determinable on the basis of their reducing action on permanganate. The amount of such reducing impurities is referred to as the permanganate time. Since the permanganate time is an important commercial test which the acid product must meet for many uses, the presence therein of such impurities is highly objectionable. Apparently the removal of minute quantities of these impurities by conventional rectification alone is difficult since the impurities distill over with the acetic acid.

Among the residual impurities which have been found to degrade the permanganate time are alkyl iodide impurities which are most likely carried over into the product stream from the catalyst solution in the reactor. Also found in the acetic acid product are various unsaturated and carbonyl impurities including crotonaldehyde and 2-ethylcrotonaldehyde. As has been previously stated, it is both difficult and costly to remove the iodides, unsaturates and carbonyl impurities from the acetic acid product by physical methods since such impurities are present in such minute amounts. Accordingly, an economical process for removing such impurities is needed.

Various methods have been suggested to purify or remove nonacidic components from carboxylic acids. For example, U.S. 4,576,683 discloses a method of separating $C_1$-$C_{10}$ aliphatic and $C_3$-$C_{10}$ olefinic carboxylic acids from mixtures with nonacids by extractive distillation using an amide as an extractant to recover an extractant-acid mixture followed by recovery of the acids from the extractant-acid mixture by rectification. The method disclosed in the patent is described as being particularly suitably applied on aqueous mixtures of formic, acetic, and/or propionic acid which mixtures contain unconverted hydrocarbons and other oxygenated compounds such as mixtures with alcohols, aldehydes, and/or ketones and which may also contain further contaminants such as effluents from the oxidation reactions. The amide extractants utilized in the patent are selected from lactams having 5 or 6 membered rings. Pyrrolidone and derivatives thereof are specifically disclosed.

U.S. 4,268,362 is concerned with providing a method of removing formaldehyde from raw acetic acid which has been formed by synthetic reactions such as oxidation of acetaldehyde, gas phase or liquid phase oxidation of butane, oxidation of petroleum naphtha or paraffins, as well as the reaction of methanol with carbon monoxide. The separation process involves treating the acetic acid in a heating zone at a temperature at about the boiling point of the acetic acid or higher, removing the heated product and delivering it to a distillation zone and operating the distillation zone so as to obtain a lower boiling fraction, a higher boiling fraction and an intermediate acetic acid fraction which will have a formaldehyde content or 300 ppm or lower.

U.S. 3,725,208 is concerned with a process for the removal of small amounts of aldehyde impurities from acrylic acids which comprises adding to the acrylic acid minor amounts of a compound selected from the group consisting of sulfuric acid, hydrazine, phenyl hydrazine, aniline, monoethanolamine, ethylene diamine and glycine and subjecting the acrylic acid mixture to distillation. Although hydrazine usually reacts exothermically with acrylic acid to form pyrrazolidone, and amines such as monoethanolamine and ethylene diamine have the properties of forming salts and aminocarboxylic acids with acrylic acid, the patentee states that it was surprising that these compounds react predominantly with aldehydes contained in acrylic acid and can remove them from the acrylic acid.

Japanese patent application 84-176729, assigned to Daicel Chemical Industries, Ltd., discloses purification of acetic acid by adding a small amount of peracetic acid to raw acetic acid, heating the mixture at 50°C. to 120°C. for about 20 hours, and thereafter subjecting the mixture to distillation.

Japanese patent application 60-222439 discloses purification of acetic anhydride produced by the ketene process in which acetic acid is thermally cracked to ketene which then combines with acetic acid through an absorption reaction to produce acetic anhydride.

An additional patent teaching improving the permanganate time of aliphatic acids is U.S. Patent 2,900,413 which discloses a heating and distillation step.

## SUMMARY OF THE INVENTION

The present invention is directed to the treatment of acetic acid and the improvement of permanganate time by subjecting the acid to hydrogenation with hydrogen in the presence of a hydrogenation catalyst. Acetic acid, such as formed and recovered from the catalytic carbonylation of methanol, may contain unsaturated and carbonyl impurities such as crotonaldehyde and 2-ethylcrotonaldehyde. By treating the acetic acid with hydrogen in the presence of a hydrogenation catalyst in accordance with the invention, the unsaturated aldehydic impurities are converted to their corresponding saturated aldehyde and/or alcohol and improvement in permanganate time is thereby achieved.

## DETAILED DESCRIPTION OF THE INVENTION

The hydrogenation treatment of the present invention is applicable to the treatment of acetic acid which has been formed by any known method such as the oxidation of natural gas or the carbonylation of dimethyl ether or methyl acetate. Preferably the treatment process of this invention is directed to acetic acid which has been produced by the carbonylation of methanol in the presence of a metal catalyst such as rhodium. The hydrogenation treatment of the present invention is particularly useful when the carbonylation reaction is catalyzed by a metal such as rhodium and a halide promoter such as an organic halide disclosed in U.S. 3,769,329. The treatment of acetic acid in accordance with the invention is more particularly useful when the acetic acid is formed by the carbonylation of methanol under the conditions such as set out in U.S. Serial No. 699,525 wherein the catalyst solution not only contains the rhodium catalyst and organic halide promoter, but also contains an alkali metal iodide salt. It has been found that unsaturated and carbonyl impurities degrade the commercial value of the acetic acid product.

In the carbonylation of methanol to acetic acid as exemplified in U.S. Serial No. 699,525, the catalyst which is employed includes a rhodium component and a halogen promoter in which the halogen is either bromine or iodine. Generally, the rhodium component of the catalyst system is believed to be present in the form of a coordination compound of rhodium with a halogen component providing at least one of the ligands of such coordination compound. In addition to the coordination of rhodium and halogen, it is also believed that carbon monoxide ligands form coordination compounds or complexes with rhodium. The rhodium component of the catalyst system may be provided by introducing into the reaction zone rhodium in the form of rhodium metal, rhodium salts and oxides, organic rhodium compounds, coordination compounds of rhodium, and the like.

The halogen promoting component of the catalyst system consists of a halogen compound comprising an organic halide. Thus, alkyl, aryl, and substituted alkyl or aryl halides can be used. Preferably, the halide promoter is present in the form of an alkyl halide in which the alkyl radical corresponds to the alkyl radical of the feed alcohol which is carbonylated. For example, in the carbonylation of methanol to acetic acid, the halide promoter will comprise methyl halide, and more preferably methyl iodide.

The liquid reaction medium employed may include any solvent compatible with the catalyst system and may include pure alcohols, or mixtures of the alcohol feedstock and/or the desired carboxylic acid and/or esters of these two compounds. The preferred solvent and liquid reaction medium for the low water carbonylation process comprises the carboxylic acid product. Thus, in the carbonylation of methanol to acetic acid, the preferred solvent is acetic acid.

Water is also added to the reaction medium, but, at concentrations well below what has heretofore been thought practical for achieving sufficient reaction rates. It is known that in rhodium-catalyzed carbonylation reactions of the type set forth in this invention, the addition of water exerts a beneficial effect upon the reaction rate (U.S. Patent No. 3,769,329). Thus, commercial operations run at water concentrations of at least 14 wt.%. Accordingly, it is quite unexpected that reaction rates substantially equal to and above reaction rates obtained with such high levels of water concentration can be achieved with water concentrations below 14 wt.% and as low as 0.1 wt.%.

In accordance with the carbonylation process most useful in the present invention, the desired reaction rates are obtained even at low water concentrations by including in the reaction medium an ester which corresponds to the alcohol being carbonylated and the acid product of the carbonylation reaction and an additional iodide ion which is over and above the iodide which is present as a catalyst promoter such as methyl iodide or other organic iodide. Thus, in the carbonylation of methanol to acetic acid, the ester is methyl acetate and the additional iodide promoter is an iodide salt, with lithium iodide being preferred. It has been found that under low water concentrations, methyl acetate and lithium iodide act as rate promoters only when relatively high concentrations of each of these components are present and that the promotion is higher when both of these components are present simultaneously. This has not been recognized in the prior art previous to disclosure of commonly assigned U.S. Serial No. 699,525. The concentration of lithium iodide used in the reaction medium of the preferred carbonylation reaction system is believed to be quite high as compared with what little prior art there is dealing with the use of halide salts in reaction systems of

4

this sort.

The carbonylation reaction may be carried out by intimately contacting the feed alcohol, which is in the liquid phase, with gaseous carbon monoxide bubbled through a liquid reaction medium containing the rhodium catalyst, halogen-containing promoting component, alkyl ester, and additional soluble iodide salt promoter, at conditions of temperature and pressure suitable to form the carbonylation product. Thus, when the feed is methanol, the halogen-containing promoting component will comprise methyl iodide and the alkyl ester will comprise methyl acetate. It will be generally recognized that it is the concentration of iodide ion in the catalyst system that is important and not the cation associated with the iodide, and that at a given molar concentration of iodide the nature of the cation is not as significant as the effect of the iodide concentration. Any metal iodide salt, or any iodide salt of any organic cation, can be used provided that the salt is sufficiently soluble in the reaction medium to provide the desired level of the iodide. The iodide salt can be a quaternary salt of an organic cation or the iodide salt of an inorganic cation. Preferably it is an iodide salt of a member of the group consisting of the metals of Group Ia and Group IIa of the periodic table as set forth in the "Handbook of Chemistry and Physics" published by CRC Press, Cleveland, Ohio, 1975-76 (56th edition). In particular, alkali metal iodides are useful, with lithium iodide being preferred. In the low water carbonylation most useful in this invention, the additional iodide over and above the organic iodide promoter is present in the catalyst solution in amounts of from 2-20, preferably 10-20 wt.%, the methyl acetate is present in amounts of from 0.5-30, preferably 2-5 wt.%, and the methyl iodide is present in amounts of from 5-20, and preferably 14-16 wt.%. The rhodium catalyst is present in amounts of from 200-1000 and preferably 300-600 ppm.

Typical reaction temperatures for carbonylation will be approximately 150-250°C, with the temperature range of about 180-220°C being the preferred range. The carbon monoxide partial pressure in the reactor can vary widely but is typically about 2-30 atmospheres, and preferably, about 4-15 atmospheres. Because of the partial pressure of by-products and the vapor pressure of the contained liquids, the total reactor pressure will range from about 15 to 40 atmospheres.

A reaction and acetic acid recovery system which can be employed, within which the present improvement is used, comprises (a) a liquid-phase carbonylation reactor, (b) a so-called "flasher", and (c) a "methyl iodide-acetic acid splitter column". The carbonylation reactor is typically a stirred vessel within which the reacting liquid contents are maintained automatically at a constant level. Into this reactor there are continuously introduced fresh methanol, sufficient water to maintain at least a finite concentration of water in the reaction medium, recycled catalyst solution from the flasher base, and recycled methyl iodide and methyl acetate from the overhead of the methyl iodide-acetic acid splitter column. Alternate distillation systems can be employed so long as they provide means for recovering the crude acetic acid and recycling to the reactor catalyst solution, methyl iodide, water and methyl acetate. In the preferred process, carbon monoxide is continuously introduced into the carbonylation reactor just below the agitator which is used to stir the contents. The gaseous feed is, of course, thoroughly dispersed through the reacting liquid by this means. A gaseous purge stream is vented from the reactor to prevent buildup of gaseous by-products and to maintain a set carbon monoxide partial pressure at a given total reactor pressure. The temperature of the reactor is controlled automatically, and the carbon monoxide feed is introduced at a rate sufficient to maintain the desired total reactor pressure.

Liquid product is drawn off from the carbonylation reactor at a rate sufficient to maintain a constant level therein and is introduced to the flasher at a point intermediate between the top and bottom thereof. In the flasher the catalyst solution is withdrawn as a base stream (predominantly acetic acid containing the rhodium and the iodide salt along with lesser quantities of methyl acetate, methyl iodide, and water), while the overhead of the flasher comprises largely the product acetic acid along with methyl iodide, methyl acetate, and water. A portion of the carbon monoxide along with gaseous by-products such as methane, hydrogen, and carbon dioxide exits the top of the flasher.

The product acetic acid drawn from the base of the methyl iodide-acetic acid splitter column (it can also be withdrawn as a side stream near the base) is then drawn off for final purification such as to remove water as desired by methods which are obvious to those skilled in the art including, most preferably, distillation. The overhead from the methyl iodide-acetic acid splitter, comprising mainly methyl iodide, methyl acetate and water, is recycled to the carbonylation reactor along with fresh methyl iodide, the fresh methyl iodide being introduced at a rate sufficient to maintain in the carbonylation reactor the desired concentration of methyl iodide in the liquid reaction medium. The fresh methyl iodide is needed to compensate for losses of methyl iodide in the flasher and carbonylation reactor vent streams.

The crude dry acetic acid product is not adequately purified since it contains residual by-products such as organic and metal iodides, unsaturates, and carbonyl impurities such as crotonaldehyde and 2-ethylcrotonaldehyde. Small amounts of these impurities ranging from about 10 to 15 ppm degrade the commercial usefulness of the acetic acid product and accordingly it has been discovered that by treating the acetic acid with hydrogen in the presence of a hydrogenation catalyst it becomes possible to obtain a good quality acetic acid product low in oxidazable impurities as evidenced by the permanganate test.

In carrying out the invention, crude or finished acetic acid which contains halide, unsaturate and carbonyl impurities is charged to a vessel, as, for example, in a stirred vessel, which contains a hydrogenation catalyst. The hydrogenation is carried out at temperatures of about 17°C to 200°C, preferably about 25°C to 120°C, in the presence of molecular hydrogen. Hydrogen is introduced into the reaction vessel to establish a hydrogen pressure ranging from about 1 to 30 atmospheres, and preferably about 5 to 50 psig. The residence time for the hydrogenation of crude or finished acetic acid under the aforesaid temperature and pressure is for a period of time, usually less than about eight hours, sufficient to saturate aldehydic impurities, such as crotonaldehyde and 2-ethylcrotonaldehyde, to the corresponding saturated aldehydes and/or alcohols. As shown hereinafter, improved permanganate time can be achieved in a batch or continuous operation such as a stirred backmixed or trickle bed hydrogenation reactor at temperatures of 25°C to 120°C with a residence time of two hours or less. At the completion of the hydrogenation reaction the acetic acid product is separated from the catalyst and may be further processed if needed. The hydrogenation reaction of this invention can be used in conjunction with the previously disclosed process for the removal of Iodine compounds as disclosed in U.S. 4,615,806.

The hydrogenation catalyst used for purposes of the invention can be any known hydrogenation catalyst which does not dissolve in acetic acid. Representative of such catalysts are platinum, palladium, rhodium, ruthenium, osmium, iridium, nickel, cobalt, etc., which may be unsupported or supported with a carrier material such as carbon, acidic clay or amorphous silica-alumina. Zeolites such as zeolites X and Y are also useful carrier materials. Raney nickel or cobalt are also useful catalysts.

Particle size of the carrier material can vary over a wide range from 0.01 to 10mm and the surface area (BET) can range from 1 to 700 $m^2/g$. Separation of the catalyst from the acetic acid product is preferably carried out by filtration.

The amount of catalyst used is preferably within the range of 0.01 to 10 weight percent, preferably 0.5 to 5.0 weight percent, based on the weight of the acetic acid.

The present invention can be more fully understood by referring to the following examples which illustrate the best mode now contemplated for carrying out the invention. In the examples the "permanganate time" is determined as follows:

One ml of an aqueous 0.1N potassium permanganate solution is added to 50 ml of acetic acid in a graduated cylinder at room temperature. The cylinder is stoppered and shaken, and a timer is immediately started to measure the time required for the purple color to change to a yellow-amber end point which is compared to a standard reference color indicating the content of oxidizable impurities such as unsaturates and carbonyl compounds. The longer time required to change to the yellow-amber color indicates a lower content of oxidizable impurities present in the acetic acid.

In the following examples, the "permanganate consumption" is determined as follows: one ml of an aqueous 0.1N potassium permanganate solution is added to 5 ml of acetic acid. This solution is shaken and then placed in a visible spectrophotometer to measure the amount of permanganate consumed in 5 minutes. The less amount of permanganate consumed in 5 minutes indicates a lower content of oxidizable impurities present in the acetic acid.


## EXAMPLES 1-7


In the following examples, crude and finished glacial acetic acid (obtained from the carbonylation of methanol to acetic acid employing a halogen promoted rhodium catalyst) were subject to treatment with hydrogen in the presence of a hydrogenation catalyst in a bath operation. In each example, 150 cc of acetic acid feed material was introduced into a 300 cc rocker bomb which contained 9 grams of hydrogenation catalyst. Unless otherwise indicated, the catalyst was 0.1 wt.% Pd/C (Girdler-G75A) or 0.5 wt.% Pd/C (Oxy-Catalyst-2785D). As shown below in Table 1, the results indicate a reduction of oxidizable compounds in acetic acid is obtained, resulting in improved quality of acetic acid as indicated from the permanganate consumption of acetic acid.

6

## TABLE 1

| BATCH HYDROGENATION EXPERIMENTS | | | | |
|---|---|---|---|---|
| RUN NO. | CATALYST | RUN DURATION (min) | KMnO4 Consumption (mM) (a) | REACTOR CONDITIONS |
| Crude HOAC | Untreated | --- | 2.0 | --------------------- |
| 1 | 0.5% Pd/C | 60 | 0.39 | 120 deg C at 100 psig |
| 2 | 0.5% Pd/C | 30 | 0.56 | 120 deg C at 100 psig |
| 3 | 0.1% Pd/C | 60 | 0.50 | 120 deg C at 100 psig |
| Finished HOAC | Untreated | --- | 0.40 | --------------------- |
| 4 | 0.1% Pd/C | 30 | 0.35 | 18 deg C at 100 psig (b) |
| 5 | 0.1 Pd/C | 30 | 0.34 | 25 deg C at 100 psig |
| 6 | 0.1% Pd/C | 30 | 0.38 | 25 deg C at 100 psig |
| 7 | 0.5% Pd/silica | 30 | 0.34 | 120 deg C at 100 psig |

(a) Quality of acetic acid is expressed in KMnO4 consumption. The KMnO4 consumption is the amount of KMnO4 consumed (millimoles per liter of acetic acid) in 5 minutes.

(b) Calsicat E--156 (0.5 wt.% Pd/C) was used for this run.

## EXAMPLES 8-18

In the following examples, finished glacial acetic acid (obtained from a low water carbonylation of methanol to acetic acid employing a halogen promoted rhodium catalyst) was subject to treatment with hydrogen in the presence of a hydrogenation catalyst in a trickle bed operation. In each example, testing was conducted in a trickle bed reactor at a temperature of 25°C (0.125 ft. ID x 8.7 ft. in length; ca. 3.1L). The amount of catalyst charged was varied for operation at several different residence times over a range of HOAC liquid mass flux. As shown below in Table 2, the results indicate a reduction of oxidizable compounds in acetic acid is obtained, resulting in improved quality of acetic acid as indicated from the permanganate consumption and permanganate time of acetic acid.

EP 0 372 993 A1

## TABLE 2

### TRICKLE BED HYDROGENATION OF FINISHED ACETIC ACID (a)

| RUN NO. | | RXN PRES (psig) | H2 FD (L/Hr) | HOAC FD (L/Hr) | RES TIME (min) | LIQUID MASS FLUX (Kg/M2/sec) | KMnO4 CONSUMPTION (b) CLP METH (min) | CCTC METH (mM) |
|---|---|---|---|---|---|---|---|---|
| Finished HOAC Untreated | | ------ | ------ | ------ | ------ | ------ | 0.25 | 0.40 |
| 8 | (c,d) | 5 | 26.1 (N2) | 10.9 | 10 | 2.8 | 0.25 | 0.40 |
| 9 | | 5 | 25.9 | 11.1 | 10 | 2.8 | 10 | 0.34 |
| 10 | | 5 | 27.0 | 10.8 | 10 | 2.8 | 4 | 0.34 |
| 11 | | 50 | 23.0 | 11.0 | 10 | 2.8 | 3 | 0.38 |
| 12 | (e) | 5 | 28.0 | 11.8 | 5 | 3.0 | 2.5 | 0.40 |
| 13 | | 5 | 28.6 | 6.3 | 10 | 1.6 | 3 | 0.38 |
| Finished HOAC Untreated | | ------ | ------ | ------ | ------ | ------ | 1.5 | 0.40 |
| 14 | (f) | 50 | 19.7 | 5.6 | 33 | 1.4 | 120 | 0.17 |
| 15 | | 50 | 51.9 | 19.4 | 10 | 5.0 | 120 | 0.16 |
| Finished HOAC Untreated | | ------ | ------ | ------ | ------ | ------ | 0.25 | 0.40 |
| 16 | | 50 | 51.4 | 18.7 | 10 | 4.8 | 20 | 0.30 |
| 17 | | 50 | 26.2 | 6.7 | 28 | 1.7 | 50 | 0.21 |
| 18 | | 5 | 50.2 | 19.7 | 9 | 6.3 | 15 | 0.31 |

(a) Catalyst testing was conducted in a trickle bed reactor(0.125 ft id x 8.7 ft in length; ca. 3.1 L) The amount of catalyst charged was varied for operation at several different residence times over a range of HOAC liquid mass flux. All runs were conducted at 25 deg C and 5 or 50 psig reactor pressure.

(b) Quality of the acetic acid is expressed in KMnO4 time and KMnO4 consumption. The KMnO4 time is the time in minutes required for 50 ml of acetic acid to consume 1 ml of 0.1 N KMnO4. The KMnO4 consumption is the amount of 0.1 N KMnO4 consumed (millimoles per liter of acetic acid) in 5 minutes by 5 ml acetic acid.

(c) For runs 8-11, 0.1 wt% Pd/low Sulfur-Granular Carbon from Calsicat (D-156M 20D-052) : 1.8 L (855 gms) charged to the reactor.

(d) Run conducted under nitrogen atmosphere (blank run) before catalyst is exposed to hydrogen atmosphere.

(e) For runs 12-13, 0.1 wt% Pd/low Sulfur-Granular Carbon from Calsicat (E-156 M 20D-052): 1.0 L (475 gms) charged to the reactor.

(f) For runs 14-18, 0.5 wt% Pd/low Sulfur-Granular Carbon from Calsicat (E-156, 65C-0388) : 3.08 L (1426 gms) charged to the reactor.

## Claims

1. A method of improving the permanganate time of acetic acid by reducing its content of impurities which are halides, unsaturates, carbonyls or mixtures thereof, characterized by subjecting the acid to hydrogenation in the presence of hydrogen and 0.01 to 10 weight percent of a hydrogenation catalyst for a period of time sufficient to hydrogenate said impurities.

2. The method of claim 1 wherein the hydrogenation is effected at 17° C to 200° C.

3. The method of claim 1 or 2 wherein the hydrogenation is effected at hydrogen pressures of 1 to 30 atmospheres.

4. The method of any of claims 1-3 wherein the hydrogenation catalyst is a platinum, palladium, rhodium, ruthenium, osmium, iridium, nickel or cobalt, catalyst.

5. The method of claim 4 wherein the temperature ranges from 25°C to 120°C at pressures ranging from 5 psig to 50 psig.

6. The method of claim 4 or 5 wherein the hydrogenation catalyst is present in an amount ranging from 0.5 to 5 weight percent based on the acetic acid.

7. The method of any of claims 1-6 wherein the acetic acid is obtained by the catalytic carbonylation of methanol.

8. A method of providing acetic acid of reduced impurity content whereby permanganate time is improved, which comprises the rhodium-catalyzed carbonylation of methanol in the presence of water and a halide promoter to produce acetic acid, and subjecting the acid to hydrogenation in accordance with any of claims 1-7.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-2 884 451 (GRAHAM)<br>* Whole document *<br>--- | 1-6 | C 07 C 53/08<br>C 07 C 51/487 |
| X | US-A-4 227 971 (ZIMMERSCHIED)<br>* Claims 1-2; column 2, lines 10-53 *<br>--- | 1-6 | |
| X | GB-A- 273 810 (DREYFUS)<br>* Claims 1-3 *<br>----- | 1-4 | |
| | | | **TECHNICAL FIELDS SEARCHED (Int. Cl.5)**<br><br>C 07 C 53/00<br>C 07 C 51/00<br>C 07 B 63/00 |

The present search report has been drawn up for all claims

| Place of search | Date of completion of the search | Examiner |
|---|---|---|
| THE HAGUE | 06-03-1990 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)